(19) Europäisches Patentamt
European Patent Office
Office européen des brevets

(11) **EP 2 135 905 A2**

(12) **EUROPEAN PATENT APPLICATION**

(43) Date of publication:
**23.12.2009 Bulletin 2009/52**

(51) Int Cl.:
*C09J 7/02* [(2006.01)]      *A61K 9/70* [(2006.01)]

(21) Application number: **09162693.7**

(22) Date of filing: **15.06.2009**

(84) Designated Contracting States:
**AT BE BG CH CY CZ DE DK EE ES FI FR GB GR HR HU IE IS IT LI LT LU LV MC MK MT NL NO PL PT RO SE SI SK TR**

(30) Priority: **16.06.2008 JP 2008156688**

(71) Applicant: **Nitto Denko Corporation**
**Ibaraki-shi, Osaka 567-8680 (JP)**

(72) Inventors:
• **Ameyama, Satoshi**
**Osaka 567-8680 (JP)**

• **Inosaka, Keigo**
**Osaka 567-8680 (JP)**
• **Nakamura, Kouji**
**Osaka 567-8680 (JP)**

(74) Representative: **Weber, Thomas**
**Patentanwälte**
**von Kreisler Selting Werner**
**Deichmannhaus am Dom**
**Bahnhofsvorplatz 1**
**50667 Köln (DE)**

(54) **Patch and patch preparation**

(57)     The present invention provides a patch capable of suppressing adhesive residue on the skin surface upon peeling off from the skin in the presence of water due to perspiration and the like, as well as easy detachment from the skin in the presence of water due to perspiration and the like. The present invention provides a patch comprising a support and an adhesive layer provided on at least one surface of the support, wherein the adhesive layer is formed by crosslinking a blend of the following component (A) to component (D): (A) a copolymer obtained by copolymerization of a monomer containing a carboxyl group, (meth)acrylate and vinylpyrrolidone as essential components; (B) a copolymer obtained by copolymerization of a monomer having a basic group as an essential component; (C) a liquid component, and (D) an acidic compound.

**Description**

**TECHNICAL FIELD OF THE INVENTION**

The present invention relates to a patch and a patch preparation both having an adhesive layer on at least one surface of a support.

**BACKGROUND OF THE INVENTION**

In recent years, various patch preparations such as a patch applicable for the protection of the skin and the like, as well as poultice, tape preparation and the like, which are applicable for the administration of a drug into the body through skin surface and the like, have been developed, and the pertinent techniques are described, for example, in the following publications.

JP-A-10-504552 discloses a patch composed of a polyester sheet and a medical pressure sensitive adhesive containing (a) a self-adhesive polyacrylate copolymer containing copolymerized (meth)acrylic acid at a given ratio, (b) a polymer containing a basic amino group and (c) a plasticizer, which is applied on the sheet. This publication indicates that the adhesive should have a minimum level of adhesiveness, which turns into higher adhesiveness in the presence of water due to perspiration and the like.

However, this publication does not disclose vinylpyrrolidone, and does not even suggest copolymerization of vinylpyrrolidone, a monomer containing a carboxyl group and (meth)acrylate.

JP-A-2000-44904 discloses a patch preparation having an adhesive layer formed by blending a copolymer obtained by copolymerizing acid 2-ethylhexyl acrylate and acrylic acid at a given ratio (Component A), an aminoalkyl methacrylate/alkyl methacrylate copolymer (Component B), a predetermined liquid component (Component C) and a drug, and crosslinking the blend (Example 3). The publication describes that such patch preparation shows well-balanced drug solubility and adhesion to the skin.

However, this publication does not disclose or suggest containing, in an adhesive layer, a particular copolymer containing a monomer having a basic group independently of a copolymer containing vinylpyrrolidone.

In addition, none of the above-mentioned references disclose or suggest addition of an acidic compound to an adhesive layer. Furthermore, none of the above-mentioned references indicate problems of suppression of adhesive residue on the skin when peeled off in the presence of water due to perspiration and the like, and falling off from the skin in the presence of water due to perspiration and the like. patent document 1: JP-T-H10-504552 patent document 2: JP-A-2000-44904

**DISCLOSURE OF THE INVENTION**

**PROBLEMS TO BE SOLVED BY THE INVENTION**

In view of the above, the present invention aims to provide a patch capable of suppressing adhesive residue on the skin when peeled off in the presence of water due to perspiration and the like, and falling off from the skin in the presence of water due to perspiration and the like.

**MEANS OF SOLVING THE PROBLEMS**

The present inventors have conducted intensive studies in an attempt to solve the aforementioned problems and found that an adhesive layer comprising an acidic compound, wherein plural kinds of particular copolymers are crosslinked by blending, is effective for maintaining cohesion force of the adhesive layer in the presence of water due to perspiration and the like, and suppressing falling off from the skin in the presence of water due to perspiration and the like. It is unpredictable that when an acidic compound is added to the adhesive layer, the adhesive layer is not easily detached from the skin even in the presence of water due to perspiration and the like, regardless of the adhesive force thereof.

Accordingly, the present invention provides the following:

(1) a patch comprising a support and an adhesive layer provided on at least one surface of the support, wherein the adhesive layer is formed by crosslinking a blend of the following component (A) to component (D):

(A) a copolymer obtained by copolymerization of a monomer containing a carboxyl group, (meth)acrylate and vinylpyrrolidone as essential components;
(B) a copolymer obtained by copolymerization of a monomer having a basic group as an essential component;

(C) a liquid component, and
(D) an acidic compound;

(2) the patch of (1), wherein the adhesive layer is crosslinked by a chemical crosslinking treatment;
(3) the patch of (1) or (2), wherein component (B) is blended in a proportion of 1 - 20 parts by weight relative to 45 parts by weight of component (A);
(4) the patch of any of (1) to (3), wherein the basic group is at least one selected from the group consisting of an optionally substituted amino group, a pyridyl group and an imidazolyl group;
(5) the patch of any of (1) to (4), wherein the adhesive layer is obtained by adding component (D) in an amount providing protons in the number of moles 0.2-fold or above that of the basic group in component (B); and
(6) a patch preparation comprising the patch of any of (1) to (5), wherein the adhesive layer contains a drug.

## BRIEF DESCRIPTIOR OF THE DRANING

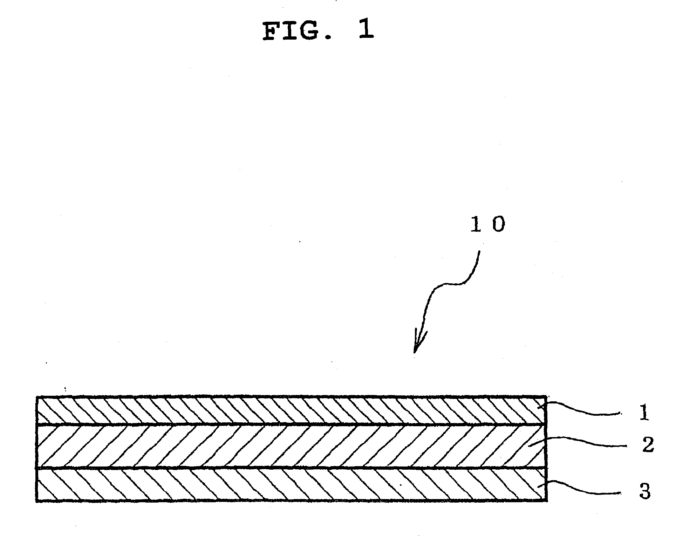

Fig. 1 shows a schematic sectional view showing one embodiment of the patch or patch preparation of the present invention.

[Explanation of symbols]

1 support
2 adhesive layer
3 release liner
10 patch or patch preparation

## EFFECT OF THE INVENTION

The patch of the present invention can maintain cohesion force of the adhesive layer in the presence of water due to perspiration and the like, since it comprises, in an adhesive layer thereof, a particular copolymer containing vinylpyrrolidone, a particular copolymer containing a monomer having a basic group, and an acidic compound in combination. Consequently, it affords remarkable effects of suppression of cohesive failure and what is called an adhesive residue on the skin surface upon peeling off from the skin during perspiration and the like, as well as resistance to easy detachment from the skin in the presence of water due to perspiration and the like.

Moreover, since the adhesive layer is efficiently crosslinked in the patch of the present invention, the adhesive layer can retain a large amount of a liquid component, thus efficiently providing a gel-like structure. Therefore, the adhesive layer affords a superior soft feeling during application to the skin and does not cause skin irritation easily during peeling.

## BEST MODE FOR CARRYING OUT THE INVENTION

As a support to be used in the present invention, one through which the copolymers constituting the adhesive layer, a liquid component, drug when to be contained and the like are less likely to permeate and lost from the back face thereof is preferable.

Specifically, a single film, a laminate film and the like, of polyester, nylon, saran, polyethylene, polypropylene, polyvinyl chloride, ethylene-ethyl acrylate copolymer, polytetrafluoroethylene, surlyn, metal foil and the like can be used.

To improve the adhesive force (anchor force) between the support and the below-mentioned adhesive layer, the support is preferably a laminate film of a non-porous plastic film and a porous film, both composed of the above-mentioned materials. In this case, the adhesive layer is preferably formed on the porous film side.

As the porous film, a film capable of improving the anchor force to the adhesive layer is employed. Specifically, paper, woven fabric, non-woven fabric, mechanically perforated sheet and the like are used. Among these, paper, woven fabric and non-woven fabric are particularly preferable from the aspects of handleability and the like.

In the patch of the present invention, an adhesive layer to be formed on at least one support of the above-mentioned support is a crosslinked structure having suitable elasticity, which is obtained by crosslinking of a blend of plural kinds of particular copolymer component (A) and component (B), liquid component (C), and acidic compound (D). It can have what is called a gel-like structure.

First, the copolymer for the above-mentioned component (A) can be obtained by copolymerizing a monomer containing a carboxyl group, vinylpyrrolidone and (meth)acrylate as essential components. The copolymer for component (A) is mainly used as a component for an adhesive layer to improve adhesiveness and compatibility with the liquid component to be added.

Examples of the monomer containing a carboxyl group for the copolymer of the above-mentioned component (A) include (meth)acrylic acid, itaconic acid, maleic acid, maleic anhydride and the like. These monomers can be used alone or as a blend of two or more kinds thereof. From the aspects of reactivity and the adhesiveness of the finished tape, acrylic acid is preferable. The proportion of these monomers is preferably 1 - 20 wt%, more preferably 1 - 10 wt%, of the copolymer of component (A), from the aspects of adhesiveness and cohesion as adhesive properties, drug releaseability when a drug is contained in an adhesive layer, reactivity during crosslinking treatment of an adhesive layer and the like.

As vinylpyrrolidone in the copolymer of the above-mentioned component (A), N-vinyl-2-pyrrolidone, methylvinylpyrrolidone and the like can be used, which may be used alone or as a blend of two or more kinds thereof. From the aspect of reactivity, N-vinyl-2-pyrrolidone is preferable. From the aspect of cohesion force, the proportion thereof is preferably 10 - 30 wt%, more preferably 19 - 30 wt%, of the copolymer of component (A).

While (meth)acrylate in the copolymer of the above-mentioned component (A) is not particularly limited, from the aspects of adhesiveness and the like, (meth)acrylic acid alkyl ester having an alkyl group having a carbon number of not less than 4 is preferably used. Specifically, (meth)acrylic acid alkyl ester having a straight or branched chain alkyl group having a carbon number of 4 to 13, such as butyl, pentyl, hexyl, heptyl, octyl, nonyl, decyl, undecyl, dodecyl, tridecyl and the like, can be used, which may be used alone or as a blend of two or more kinds thereof.

From the aspect of adhesiveness at ambient temperature, (meth)acrylic acid alkyl ester wherein the alkyl group is butyl, 2-ethylhexyl or cyclohexyl is more preferable, and (meth)acrylic acid alkyl ester wherein the alkyl group is 2-ethylhexyl is most preferable. From the aspect of adhesiveness, the proportion of these monomers can be set to any level as long as it falls within the range of preferably 30 - 89 wt%, more preferably 60 - 80 wt%, of the copolymer of component (A).

The above-mentioned (meth)acrylic acid alkyl ester is not limited to those recited above as examples, and it is obvious that an ester compound of a group other than alkyl group, (meth)acrylic acid alkyl ester having an alkyl group with a carbon number of 1 to 3 and (meth)acrylic acid alkyl ester having an alkyl group with a carbon number of not less than 14 may be used in combination, as long as the effect of the present invention can be exhibited.

In the present invention, the copolymer of component (B) is used as a component for improving the adhesiveness of an adhesive layer, and further, improving drug solubility when an adhesive layer contains a drug. The above-mentioned copolymer component (B) can be obtained by copolymerization of, as an essential component, a monomer having a basic group.

In the copolymer of the above-mentioned component (B), as monomer containing a basic group, a monomer containing a group having a basic nitrogen atom is preferable from the aspect of handleability such as reactivity and the like. Examples of the group having such basic nitrogen atom include an optionally substituted amino group (particularly alkylamino group), a pyridyl group, an imidazolyl group and the like. Examples of the monomer having a group containing a basic nitrogen atom include acrylic monomer having thereon a side chain or a vinyl monomer and the like. More specifically, mono or dialkylamino(meth)acrylate having an alkyl group with a carbon number of 1 to 4, such as aminoethyl (meth)acrylate, dimethylaminoethyl(meth)acrylate, dimethylaminopropyl(meth)acrylate, dimethylaminobutyl(meth)acrylate and the like, vinylpyridine, vinylimidazole and the like are preferable. Of these, from the aspect of handleability such as reactivity and the like, a monomer having a basic group is preferably dimethylaminoethyl(meth)acrylate. These monomers having a basic group can be used alone or in a blend of two or more kinds thereof. The proportion of these monomers is preferably 20 - 70 wt%, more preferably 30 - 60 wt%, of the copolymer of component (B), from the aspects of compatibility with the copolymer of component (A), adhesive property to be maintained and the like.

In addition, the copolymer of the above-mentioned component (B) preferably comprises (meth)acrylate to adjust adhesiveness of the adhesive layer. While the (meth)acrylate in the copolymer of the above-mentioned component (B) is not particularly limited, use of (meth)acrylic acid alkyl ester having an alkyl group with a carbon number of one or more is preferable from the aspects of adhesiveness and the like. Specifically, (meth)acrylic acid alkyl ester wherein the alkyl group is a straight chain or branched alkyl group having a carbon number of 1 to 10 such as methyl, ethyl, propyl, butyl, pentyl, hexyl, heptyl, octyl, nonyl, decyl and the like can be used alone or in a combination of two or more kinds thereof. The proportion of the (meth)acrylate (as a total amount when plural kinds are used) is preferably 30 - 80 wt%, more preferably 40 - 70 wt%, of copolymer component (B), from the aspects of compatibility with copolymer component (A), adjustment of adhesiveness and the like.

The copolymers of the above-mentioned component (A) and component (B) may be each copolymerized with any amount of other copolymerizable monomers as necessary, as long as the property of each component in the present invention can be maintained. The copolymerization reaction can be performed according to a method known per se, which is, for example, a method including reacting the above-mentioned monomer and a polymerization initiator (e.g., benzoyl peroxide, 2,2'-azobisisobutyronitrile etc.) in a solvent (ethyl acetate etc.) at 50 to 70°C for 5 to 48 hr.

Among the copolymers of the above-mentioned component (A) and component (B), most preferred as the copolymer of component (A) is a copolymer obtained by copolymerizing 60 - 80 wt% of 2-ethylhexyl acrylate, 19 - 30 wt% of N-vinyl-2-pyrrolidone, and 1 - 10 wt% of acrylic acid as essential components, most preferred as the copolymer of component (B) is a copolymer obtained by copolymerizing 30 - 60 wt% of dimethylaminoethyl methacrylate with 15 - 40 wt%, preferably 20 - 40 wt%, of methyl methacrylate and 15 - 40 wt%, preferably 20 - 40 wt%, of butyl methacrylate as essential components, and the like, in view of comfortableness during adhesion and the balance of drug solubility when an adhesive layer contains a drug.

While the proportion of the total weight of component (A) and component (B) relative to the total weight of the adhesive layer is not particularly limited, it is preferably 30 - 65 wt%, more preferably 35 - 60 wt%, of the total weight of the adhesive layer to impart sufficient adhesiveness to an adhesive layer. While the proportion of component (A) and component (B) is not particularly limited, 1 - 20 parts by weight, more preferably 1.5 - 12.5 parts by weight of component (B) relative to 45 parts by weight, of component (A) is preferably blended. The patch of the present invention shows sufficient adhesive force, and further, sufficient drug solubility when a drug is contained when component (B) is not less than 1 part by weight, and maintains suitable adhesive force and suppresses skin irritation upon detachment when component (B) is not more than 20 parts by weight.

The liquid component as component (C) to be contained in an adhesive layer together with the above-mentioned component (A) and component (B) in the present invention is uniformly dissolved and dispersed in the adhesive layer, plasticizes the crosslinked adhesive layer into a gel-like state, and achieves a soft feeling thereof. In other words, pain and skin irritation due to an adhesive force (skin adhesive force), which is produced when an acrylic adhesive tape and a transdermal absorption preparation of the present invention are peeled off from the skin surface, can be reduced in the present invention by adding component (C) to cause crosslinking gellation. Moreover, since an adhesive layer is plasticized as mentioned above, when a drug is contained as a patch preparation, the drug acquires good free diffusability, which in turn improves releaseability onto the skin surface (transfer to the skin).

Component (C) is not particularly limited as long as it has compatibility with the above-mentioned component (A) and component (B), and has a plasticizing action on an adhesive layer. From the aspect of the compatibility, an organic liquid component is preferable. Examples thereof include glycols such as ethylene glycol, diethylene glycol, triethylene glycol, propylene glycol, polyethylene glycol, polypropylene glycol and the like, fats and oils such as olive oil, castor oil, squalene, lanolin and the like, organic solvents such as ethyl acetate, ethyl alcohol, dimethyldecyl sulfoxide, methyloctyl sulfoxide, dimethyl sulfoxide, dimethylformamide, dimethylacetamide, dodecylpyrrolidone, isosorbitol and the like, liquid surfactant, plasticizers such as tributyl acetylcitrate, diisopropyladipate, phthalic acid ester, diethyl sebacate, triethyl citrate, tributyl acetylcitrate and the like, hydrocarbons such as liquid paraffin and the like, ethoxylated stearyl alcohol, glycerol fatty acid ester, isopropyl myristate, isotridecyl myristate, ethyl laurate, N-methylpyrrolidone, ethyl oleate, oleic acid, diisopropyl adipate, isopropyl palmitate, 1,3-butanediol and the like. These may be used alone or as a blend of two or more kinds thereof.

Of the liquid components of the above-mentioned component (C), preferable organic liquid components include fatty acid ester, glycerol fatty acid ester (particularly mono, di or triglyceride), tributyl acetylcitrate.

These fatty acid ester and glycerol fatty acid ester may be any as long as they exhibit an action to plasticize an adhesive layer. To maintain compatibility with the aforementioned acrylic copolymer and to prevent volatilization in the heating step to prepare a patch, one comprising fatty acid having the below-mentioned carbon number is preferable.

In addition, to maintain preservation stability of a patch, the aforementioned fatty acid ester and glycerol fatty acid ester is preferably comprised of a fatty acid without a double bond in a molecule. In the case of the below-mentioned patch preparation, moreover, a drug exceeding saturation solubility may crystallize in the preparation when the drug content per unit area is high. Accordingly, fatty acid ester and glycerol fatty acid ester to be added are preferably those that do not adversely influence crystallization and precipitation rate of the drug, and the appearance and preservation stability of the obtained preparation.

As the fatty acid ester to be used, therefore, a fatty acid ester composed of higher fatty acid preferably having a carbon number of 12 - 16, more preferably 12 - 14, and lower monadic alcohol preferably having a carbon number of 1 - 4 is preferably employed.

Examples of the higher fatty acid preferably include lauric acid (C12), myristic acid (C14) and palmitic acid (C16), particularly myristic acid. Examples of the lower monadic alcohol include methyl alcohol, ethyl alcohol, propyl alcohol and butyl alcohol. They are not limited to straight chain alcohols and may be branched alcohols. Preferably, isopropyl alcohol is used. Accordingly, the most preferable fatty acid ester is isopropyl myristate.

On the other hand, as the glycerol fatty acid ester, glycerides composed of higher fatty acid having a carbon number of 8 - 10 and glycerol is preferable. Preferable examples of the higher fatty acid include caprylic acid (octanoic acid, C8), pelargonic acid (nonanoic acid, C9) and capric acid (decanoic acid, C10). Preferable examples of the glycerol fatty acid ester include glyceride composed of caprylic acid and glycerol (e.g., monoglyceride caprylate, diglyceride caprylate and triglyceride caprylate).

The proportion of the component (C) is preferably 0.5 - 1.5 parts by weight, more preferably 0.7 - 1.5 parts by weight, per 1 part by weight of the total amount of the above-mentioned component (A) and component (B). When the proportion of component (C) is within this range, practical skin adhesiveness and low skin irritation can be afforded, and the drug releaseability (skin transferability) can be sufficiently ensured in the below-mentioned patch preparation.

An acidic compound as component (D) to be added to an adhesive layer together with the above-mentioned component (A), component (B) and component (C) in the present invention are uniformly dissolved and dispersed in the adhesive layer and make it difficult for the patch to be peeled off from the skin in the presence of water due to perspiration and the like. The mechanism is not necessarily clear, but it is assumed that the crosslinking structure due to the basic group in component (B) is slightly weakened by the acidic compound of component (D), which relates to placing priority on other crosslinking structures.

As such component (D), any acidic organic or inorganic compound can be used and is not particularly limited. In consideration of safety and use performance, however, a pharmaceutically acceptable acidic compound is preferable. Specific examples include organic acids such as acetic acid, lactic acid, benzoic acid, salicylic acid, citric acid, tartaric acid, succinic acid, fumaric acid, maleic acid, mesylic acid, propionic acid and the like, inorganic acids such as hydrochloric acid, sulfuric acid, nitric acid and the like. When desired, these acidic compounds may be added to a monomer containing a basic group in the above-mentioned component (B).

While the amount of component (D) is not particularly limited, component (D) is desirably added in an amount that provides protons in the number of moles 0.2-fold or more, preferably 0.2- to 1.6-fold or more, more preferably 0.6- to 1.4-fold, that of the basic group in component (B), preferably a group containing a basic nitrogen atom, for example, amino group, pyridyl group or an imidazolyl, each of which is optionally substituted. In the present specification, when component (D) is an inorganic compound, an amount that provides protons in a given number of moles means an amount that stoichiometrically contains ionically-bonded protons in a given number of moles. When component (D) is an organic compound, an amount that provides protons in a given number of moles means an amount that stoichiometrically contains proton-donating groups in a given number of moles. When the amount of component (D) is within the above ranges, the patch is hardly detached from the skin in the presence of water due to perspiration and the like, and a decrease in the pH of an adhesive layer and accompanying skin irritation can be suppressed.

The number of moles of the basic group in component (B) in the present specification is calculated by the following calculation formula:

$$\text{number of moles of basic group in component (B)} = \text{amount of component (B) [g]} \times (\text{weight ratio of basic group in component (B) [wt\%]}/100)/\text{chemical formula weight of basic group.}$$

When the basic group contains a basic nitrogen atom, the weight ratio [wt%] of the basic group in component (B) in the present specification is calculated based on the weight ratio [wt%] of nitrogen in component (B) as measured by the nitrogen quantification method (Semimicro-Kjeldahl Method) in the Japanese Pharmacopoeia and the atomic weight of nitrogen.

In the present invention, as for the acidic compound of component (D), a monovalent acidic substance (e.g., 1 mol of hydrochloric acid) provides 1 mol of proton, and a divalent acidic substance (e.g., 1 mol of tartaric acid) provides 2 mol of protons. Similarly, 1 mol of a trivalent or more acidic substance provides 3 or more mol of protons.

The adhesive layer in the patch of the present invention contains the above-mentioned component (A), component (B), component (C) and component (D) as essential components. When these components are to be solved or mixed, they need to be compatible with each other. Thus, a mixed solvent of an amphiphilic solvent compatible with both water and oil, such as isopropyl alcohol and methanol, tetrahydrofuran, acetone and the like, and a hydrophobic solvent, such as ethyl acetate and the like, that dissolves a copolymer, is preferably used for mixing.

In the present invention, blending is performed as mentioned above, and preferably, an adhesive layer is crosslinked to form what is called a gel-like state, thereby suppressing diffluence of the contained liquid component, and further conferring a cohesion force to the adhesive layer.

Crosslinking is performed by physical crosslinking by exposure to radiation such as UV irradiation, electron beam irradiation and the like, chemical crosslinking and the like. To reduce an influence on adhesive layer components, chemical crosslinking is preferable. Specifically, chemical crosslinking using crosslinking agents such as polyisocyanate compounds, organic peroxides, organic metal salts, metal alcoholates, metal chelate compounds, multifunctional compounds and the like are employed.

Of these crosslinking agents, to effectively ensure the cohesion force of an adhesive layer in the presence of water, a chelate compound, particularly, an aluminum chelate compound, is preferable. Such crosslinking agents are free of a thickening phenomenon of the solution up to coating and drying, and are extremely superior in workability. The amount of the crosslinking agent to be blended in this case is preferably about 0.01 - 2 parts by weight relative to 100 parts by weight of the total weight of the polymer of component (A).

Since the patch preparation of the present invention protects the adhesive face of an adhesive layer before use, a release liner is preferably laminated on the adhesive face.

The release liner is not particularly limited as long as it ensures sufficient easy-to-release property. Examples thereof include films of polyester, polyvinyl chloride, polyvinylidene chloride, polyethylene terephthalate and the like, paper such as high quality paper, glassine paper and the like, laminate films of polyolefin and paper such as high quality paper, glassine paper and the like, whose face to be in contact with an adhesive layer underwent a peeling treatment by applying a silicone resin, a fluororesin and the like. The thickness of the release liner is generally 10 - 200 μm, preferably 25 - 100 μm.

As the release liner in the present invention, one made of a polyester (particularly, polyethylene terephthalate) resin is preferable from the aspects of barrier property and cost. In this case, one having a thickness of about 25 - 100 μm is more preferable in view of handleability.

A patch preparation can be obtained by impregnating an adhesive layer of a patch as mentioned above with a drug. The drug here is not particularly limited, but a drug that can be administered to a mammal such as human and the like through the skin, i.e., transdermally absorbable drug, is preferable. Where necessary, two or more kinds of drugs may be contained.

The proportion of these drugs can be appropriately determined according to the drug species and administration purpose. In consideration of the release of an amount effective for the treatment or prophylaxis, economic aspect and adhesiveness to the skin, it is preferably about 1 - 40 wt% of the total weight of the adhesive layer.

The patch and patch preparation of the present invention are produced, for example, in the following manner: starting materials such as copolymers, a liquid component and, where necessary, a drug, a crosslinking agent and the like are dissolved or dispersed in a solvent, the obtained solution or dispersion is applied on at least one surface of a support, which is dried to form an adhesive layer on the surface of the support, and a release liner is formed. Alternatively, the above-mentioned solution or dispersion is applied on at least one surface of a release liner for protection, which is dried to form an adhesive layer on the surface of the release liner, and a support is adhered to the adhesive layer. It is preferable to further include a curing step to promote crosslinking of the adhesive layer.

## Examples

The present invention is explained in more detail in the following by referring to Examples, which are not to be construed as limitative. In the following description, parts and % mean parts by weight and wt%, respectively.

(Preparation of copolymer solution)

As component (A), 2-ethylhexyl acrylate (72 wt%), N-vinyl-2-pyrrolidone (25 wt%), and acrylic acid (3 wt%) were copolymerized to give acrylic copolymer (a).

As component (B), dimethylaminoethyl methacrylate (50 wt%), methyl methacrylate (25 wt%) and butyl methacrylate (25 wt%) were copolymerized to give a copolymer.

1. Patch

(Example 1)

To a solution of acrylic copolymer (a) as component (A) (solid content 45 parts) was added a solution of the above-mentioned component (B) (average molecular weight; 150,000, weight ratio of basic nitrogen atom; 4.3%) in ethyl acetate (solid content 2 parts). Thereto was added 0.18 part of methanolic hydrochloric acid containing of hydrochloric acid as component (D) that provides protons in the number of moles 0.8-fold that of the basic nitrogen in 2 parts of component (B). Thereto was further added 52.84 parts of isopropyl myristate (hereinafter to be referred to as "IPM") as component (C). Ethylacetoacetate aluminum diisopropylate was added thereto as a crosslinking agent, suitable amounts of isopropanol and ethyl acetate were added to adjust the concentration, and the mixture was stirred in a high-speed mixer to give a uniform adhesive solution. The crosslinking agent was added in a proportion of 0.3 part per 100 parts of the solid content of acrylic copolymer (a). The obtained adhesive solution was applied to a 75 μm-thick polyester release sheet such that the thickness after drying would be 80 μm, and dried to give a crosslinked gel-like adhesive layer.

The adhesive layer prepared above was laminated on the surface of a non-woven fabric as a support, which was prepared by adhesion laminating a polyester non-woven fabric (8 g/m$^2$ fabric weight) on one surface of a 2 μm-thick polyester film, and the laminate was aged with heating at 60°C for 48 hr to give a patch of the present invention.

The amount of hydrochloric acid as component (D) that provides protons in the number of moles 0.8-fold that of the basic nitrogen in 2 g of component (B) was calculated to be 0.18 g by the following calculating formula.

The amount of component (D) [g]={( amount of component (B) [g]×(amount of component (B) [g]×weight ratio [wt%] of basic nitrogen in component (B)/100/atomic weight of nitrogen)×0.8×molecular weight of component

$$(D)=2×4.3/100×0.8×36.46=0.18 \; [g]$$

Therefore, 0.18 part of component (D) was added relative to 2 parts of component (B).

(Example 2)

The patch of Example 2 was prepared in the same manner as in Example 1 except the following items: 5 parts of component (B) was used instead of 2 parts thereof; 0.45 part of hydrochloric acid was used as component (D) that provides proton in the number of moles corresponding to 0.8-fold that of the basic group in 5 parts of component (B); component (C) was decreased in an amount comparable to the increase in component (B) and component (D).

(Example 3)

The patch of Example 3 was prepared in the same manner as in Example 1 except the following items:

10 parts of component (B) was used instead of 2 parts thereof; 0.90 part of hydrochloric acid was used as component (D) that provides proton in the number of moles corresponding to 0.8-fold that of the basic group in 10 parts of component (B); component (C) was decreased in an amount comparable to the increase in component (B) and component (D).

(Example 4)

The patch of Example 4 was prepared in the same manner as in Example 2 except the following items:

0.67 part of hydrochloric acid was used as component (D) that provides proton in the number of moles corresponding to 1.2-fold that of the basic group in component (B);
component (C) was decreased in an amount comparable to the increase in component (B).

(Example 5)

The patch of Example 5 was prepared in the same manner as in Example 2 except the following items:

0.22 part of hydrochloric acid was used as component (D) that provides proton in the number of moles corresponding to 0.4-fold that of the basic group in component (B);
component (C) was decreased in an amount comparable to the increase in component (B).

(Example 6)

The patch of Example 6 was prepared in the same manner as in Example 2 except the following items:

0.11 part of hydrochloric acid was used as component (D) that provides proton in the number of moles corresponding to 0.2-fold that of the basic group in component (B);
component (C) was decreased in an amount comparable to the increase in component (B).

(Example 7)

The patch of Example 7 was prepared in the same manner as in Example 2 except the following items:

1.10 parts of hydrochloric acid was used as component (D) that provides proton in the number of moles corresponding to 1.2-fold that of the basic group in component (B);
component (C) was decreased in an amount comparable to the increase in component (B).

(Comparative Examples 1 - 3)

The patches of Comparative Examples 1 - 3 were prepared in the same manner as in Examples 1 - 3 except that component (D) was not added and component (D) was replaced with the same amount of component (C).

(Experimental Example)

<Cohesion force of adhesive layer in the presence of water>

A sample was adhered onto the upper inner arm of 5 volunteers, and peeled off 1 to 2 hours after perspiration or bathing. Adhesive residue at that time was evaluated based on the following criteria and averaged.
Criteria:

1 No adhesive residue
2 Slight adhesive residue
3 Clear adhesive residue

The adhesive layer of a sample with a large amount of adhesive residue was evaluated to have low cohesion force in the presence of water.

<Easiness of peeling off during perspiration and bathing>

A sample was adhered onto the upper inner arm of 5 volunteers, and easiness of peeling off of the sample during perspiration and bathing was evaluated based on the following 3-level criteria and averaged.
Criteria:

1 does not peel off without force
2 peeled off with a small force
3 peeled off even without force

<Adhesive force>

A band-like strip (width 24 mm) of each preparation sample was adhered to a bakelite board, pressed by one reciprocation of a roller with a load of 300 g and peeled off in a 180 degree direction at a rate of 300 mm/min, and the adhesive force (release force) was measured.

<Gel fraction>

Each sample was cut into 10 cm$^2$, and the weight ($W_1$) [g] of the adhesive layer was measured. The sample was immersed in 100 ml of ethyl acetate for 24 hr, and ethyl acetate was exchanged. This operation was repeated three times and the solvent-soluble part was extracted. The sample was taken out and dried, and the weight ($W_2$) [g] of the adhesive layer was measured, based on which the gel fraction was calculated from the following formula.

```
Gel fraction (%)=(W₂×100)/(W₁×α/β)
```

```
α [g] = weight [g] of component (A) + weight [g] of
crosslinking agent
```

$$\beta \,[g] = \text{weight [g] of component (A) + weight [g] of component (B) + weight [g] of component (C) + (weight [g] of component (D)) + weight [g] of crosslinking agent + weight [g] of drug}$$

when it is contained

The sample with high gel fraction was evaluated to have a sufficiently crosslinked adhesive layer.

The results are shown in Table 1.

Table 1

| | component (B) (solid content, parts) | component (C) IPM (parts) | kind of component (D)/amount (parts) | cohesion force of adhesive layer in the presence of water | easiness of peeling off during perspiration and bathing | adhesive force (N/24 mm) | gel fraction (%) |
|---|---|---|---|---|---|---|---|
| Example 1 | 2 | 52.82 | hydrochloric acid/0.18 | 1 | 1.0 | 1.3 | 83 |
| Example 2 | 5 | 49.55 | hydrochloric acid/0.45 | 1 | 1.0 | 1.3 | 82 |
| Example 3 | 10 | 44.10 | hydrochloric acid/0.90 | 1 | 1.0 | 1.4 | 84 |
| Example 4 | 5 | 49.33 | hydrochloric acid/0.67 | 1 | 1.0 | 1.3 | 82 |
| Example 5 | 5 | 49.78 | hydrochloric acid/0.22 | 1 | 1.2 | 1.5 | 84 |
| Example 6 | 5 | 49.89 | hydrochloric acid/0.11 | 1 | 1.6 | 1.7 | 86 |
| Example 7 | 5 | 48.90 | acetic acid/1.10 | 1 | 1.4 | 1.7 | 76 |
| Comparative Example 1 | 2 | 53 | - | 1 | 2.2 | 1.8 | 84 |
| Comparative Example 2 | 5 | 50 | - | 1 | 2.2 | 2.0 | 89 |
| Comparative Example 3 | 10 | 45 | - | 1 | 2.8 | 2.6 | 99 |
| Component (A) (solid content) 45 parts | | | | | | | |

As is clear from Table 1, the patches of the Examples were sufficient in the cohesion force in the presence of water, adhesive force and gel fraction of the adhesive layer. In addition, the patches of the Examples were not easily peeled off in the presence of water due to perspiration or bathing.

In contrast, although the adhesive force in the Comparative Examples was higher than that in the Examples, the patches of the Comparative Examples were more easily peeled off than the patches of the Examples in the presence of water due to perspiration or bathing. In Examples 1 - 4, the patches were particularly less apt to peel off in the presence of water due to perspiration or bathing.

2. Patch preparation

In the same manner as in Examples 1 - 6 except that 1 part of 44.10 - 52.82 parts of IPM as component (C) is replaced by a drug (indomethacin), a patch preparation of the present invention is prepared. The patch preparation of the present invention shows less adhesive residue upon peeling, like the patch of the present invention, and has a sufficient skin adhesive force even during perspiration or bathing.

**Claims**

1. A patch comprising a support and an adhesive layer provided on at least one surface of the support, wherein the adhesive layer is formed by crosslinking a blend of the following component (A) to component (D):

   (A) a copolymer obtained by copolymerization of a monomer containing a carboxyl group, (meth)acrylate and vinylpyrrolidone as essential components;
   (B) a copolymer obtained by copolymerization of a monomer having a basic group as an essential component;
   (C) a liquid component, and
   (D) an acidic compound.

2. The patch of claim 1, wherein the adhesive layer is crosslinked by a chemical crosslinking treatment.

3. The patch of claim 1, wherein component (B) is blended in a proportion of 1 - 20 parts by weight relative to 45 parts by weight of component (A).

4. The patch of claim 1, wherein the basic group is at least one selected from the group consisting of an optionally substituted amino group, a pyridyl group and an imidazolyl group.

5. The patch of claim 1, wherein the adhesive layer is obtained by adding component (D) in an amount providing protons in the number of moles 0.2-fold or above that of the basic group in component (B).

6. A patch preparation comprising the patch of any of claims 1 to 5, wherein the adhesive layer comprises a drug.

FIG. 1

1 0

1

2

3

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 10504552 A
- JP 2000044904 A
- JP H10504552 T